(19) Europäisches Patentamt
European Patent Office
Office européen des brevets

(11) **EP 2 630 975 A1**

(12) **EUROPÄISCHE PATENTANMELDUNG**

(43) Veröffentlichungstag:
**28.08.2013 Patentblatt 2013/35**

(51) Int Cl.:
*A61L 9/12* *(2006.01)*    *A61L 9/01* *(2006.01)*
*G01N 31/22* *(2006.01)*

(21) Anmeldenummer: **12008256.5**

(22) Anmeldetag: **11.12.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **27.02.2012 EP 12001276**

(71) Anmelder: **Symrise AG**
**37603 Holzminden (DE)**

(72) Erfinder:
• **Müller, Dirk**
  **37603 Holzminden (DE)**
• **Rube, Jennifer**
  **37603 Holzminden (DE)**
• **Wiedemann, Jörn**
  **37603 Holzminden (DE)**

(54) **Stabile Raumdüfte mit Nutzungsendpunktanzeige**

(57) Es werden Mischungen offenbart, enthaltend
a. mindestens einen solvatochromen Farbstoff,
b. mindestens ein Lösungsmittel,
c. mindestens einen Duftstoff der ausgewählt ist aus der Gruppe, die gebildet wird von ungesättigten Duftstoffen, Ketonen, Ethern, Moschusduftstoffen und Alkoholen sowie gegebenenfalls
d. mindestens ein Stabilisierungsmittel.

Die erfindungsgemäßen Mischungen dienen bevorzugt der Beduftung von Räumen und weisen eine Nutzungsendpunktanzeige auf.

EP 2 630 975 A1

**Beschreibung**

Gebiet der Erfindung

Die Erfindung befindet sich auf dem Gebiet der Duftstoffe und betrifft spezielle Mischungen, die Duft- und Farbstoffe enthalten, ein Verfahren zur Nutzungsendanzeige von Beduftungssystemen sowie eine Vorrichtung, die diese Mischungen enthält.

Stand der Technik

Zur Beduftung und Erfrischung von Räumen werden neben Versprühsystemen insbesondere Verdunstungssysteme verwendet. Diese Verdunstungssysteme bestehen in der Regel aus Trägermaterialien, sehr einfachen Behältern sowie sehr speziellen Behältern mit Hilfsmitteln, aus denen die Duftstoffe an die Raumluft abgegeben werden. Seit langer Zeit ist man bestrebt, dem Verbraucher durch ein visuelles Signal anzuzeigen, dass der Vorrat der Duftstoffe verbraucht ist.

In der US 4,128,508 löst man diese Aufgabe durch ein Indikatorsystem enthaltend einen pH-Indikator und eine langsam verdunstende Säure oder Base. Durch die Änderung des pH-Wertes der Duftstoffmischung zeigt die veränderte Farbe des pH-Indikators das Nutzungsende an. WO 03/031966 A1 beschreibt ein System enthaltend flüchtige Farben. Durch das Verdunsten der flüchtigen Farbe wird eine Farbänderung erzielt, die den Verbrauch der Duftstoffe signalisiert. Nachteilig an beiden Systemen ist, dass die Farbänderung nicht durch die Duftstoffe selbst hervorgerufen wird, sondern durch Hilfsstoffe. Es ergibt sich zur Erzielung einer verlässlichen Nutzungsendpunktanzeige die Notwendigkeit, die Duftstoffe so auszuwählen, dass diese genau so schnell verdunsten wie die Hilfsstoffe. Die Darstellung eines kreativen und komplexen Duftes wird dadurch unmöglich oder zumindest erheblich erschwert.

GB 2444702 A offenbart nicht wässrige Mischungen enthaltend Halochrome, Duftstoffe, nicht flüchtige Säuren oder Basen sowie hochpolare, schwerflüchtige Lösungsmittel. Nachteilig an einem solchen System sind einerseits die verwendeten Halochrome, welche über sehr reaktive Atome, beispielsweise Halogenatome oder reaktive Gruppen, beispielsweise Aminogruppen, verfügen. Daneben ist die Anwesenheit von Säuren oder Basen erforderlich, welche ein günstiges Milieu für Reaktionen schaffen. Infolge dieser ungünstigen Bedingungen neigen diese Systeme zu vielfältigen Reaktionen, so dass sowohl die Duftstoffe als auch die Halochrome abgebaut werden und bereits nach kurzer Zeit eine Verringerung der Geruchsintensität bzw. die Bildung von Fehlgerüchen als auch ein Verblassen der Farbe zu beobachten sind. Für eine wirtschaftliche Nutzung ist jedoch eine sehr lange Lagerstabilität der Duftstoffsysteme erforderlich, beispielsweise wegen langer Lagerzeiten beim Vertrieb der Raumdüfte.

DE 102009058078 offenbart ein Mottenschutzsäckchen enthaltend unter anderem den Duftstoff Lavandinöl Abrialis, das Lösungsmittel Dipropylenglykol und den solvatochromen Farbstoff Nilblau. Die Farbänderung der Nutzungsendpunktanzeige soll vermutlich insbesondere auf der Verdunstung des Lösungsmittels Dipropylenglycol beruhen, nicht jedoch auf der Verdunstung des Duftstoffs Lavandinöl Abrialis. Es ist zudem anzunehmen, dass der Farbumschlag von blau nach weiß durch einen Abbau des Nilblaus erfolgt, da die Färbung des Nilblau je nach Umgebungsmedium in der Regel von grün, blau bis hin zu rot variiert und festes Nilblau dunkelgrün, blau oder schwarz erscheint. (vgl. http: //de.wikipedia.org/wiki/Nilblau#Nilblau) Es bleibt nämlich unklar, ob es sich bei der weißen Färbung um die Farbe des ggf. weißen Mottensäckchens handelt. Die Anzeige des Nutzungsendes durch eine weiße Farbgebung ist außerdem mit einer geringen Signalwirkung verbunden. Die Stabilität der Formulierung unter Lichteinwirkung ist nicht offenbart, jedoch werden die Duftsysteme gerade im Haushalt oder im Supermarkt hohen Lichtintensitäten ausgesetzt, was zum beschleunigten Abbau der Farb- und Duftstoffe führt. Die Duftsysteme verblassen folglich durch die Bestrahlung mit hohen Lichtintensitäten beschleunigt oder / und die Geruchsintensität nimmt beschleunigt ab oder es treten beschleunigt unerwünschte Fehlgerüche auf.

EP0309173 offenbart Nutzungsendpunktanzeigen enthaltend Lösungsmittel, Indikatorfarbe, bevorzugt ausgewählt aus der Gruppe der Xanthene, sowie Duftstoffe. Die Nutzungsendpunktanzeigen weisen einen sehr gut erkennbaren Farbwechsel auf und sind selbst in heißen Räumen bei einer Temperatur von 38°C über einige Tage stabil. Jedoch offenbart dieses Dokument keine Duftstoffsysteme mit Nutzungsendpunktanzeige, die auch bei Bestrahlung mit hohen Lichtintensitäten ausreichend lange stabil sind.

WO2009044363 offenbart Kompositionen zur Anwendung auf Keratinmaterialien, wobei die Kompositionen ein holographisches Pigment enthalten. Nachteilig an Pigmenten ist ihre Unlöslichkeit und deshalb neigen solche Kompositionen, sofern man diese beispielsweise in Flüssigkeiten mit niedriger Viskosität einsetzen würde, zum Entmischen und folglich zu einem ungleichmäßig gefärbten Produkt.

WO2011042222 offenbart Duftabgabesysteme mit Aktivierungs- und/oder Verbrauchsanzeige, enthaltend ggf. solvatochrome Farbstoffe. Es wird dort nicht offenbart, wie sich sehr stabile und insbesondere unter starker Lichtbestrahlung stabile Duftabgabesysteme darstellen lassen.

Die Aufgabe der vorliegenden Erfindung hat daher in erster Linie darin bestanden, Duftstoffsysteme mit Nutzungsendpunktanzeige zur Verfügung zu stellen, die die eingangs geschilderten Nachteile des Stands der Technik vermeiden. Die Systeme sollten sich dadurch auszeichnen, dass die Endpunktanzeige nicht durch Verringerung der Konzentration eines Hilfsstoffs bewirkt wird, sondern möglichst durch die Verdunstung der Duftstoffe. Zudem bestanden weitere Teilaufgaben darin, besonders lagerstabile Systeme zur Verfügung zu stellen, darunter insbesondere Systeme, die eine besonders hohe Stabilität bei der Bestrahlung mit Licht aufweisen. Die verbesserte (Licht-)Stabilität betrifft neben den Farbstoffen auch die (Licht-)Stabilität der Duftstoffe, so dass insbesondere nach stärkerer Bestrahlung der erfindungsgemäßen Raumdüfte mit Licht sowohl ein stark wahrnehmbares Farbänderungssignal als auch ein deutlich wahrnehmbarer Wohlgeruch wahrgenommen werden kann. Ferner sind die erfindungsgemäßen Duftstoffsysteme bevorzugt homogen gefärbt oder /und weisen bevorzugt als Signalfarbe des Nutzungsendes eine von Weiß verschiedene Farbe auf.

**Beschreibung der Erfindung**

Die Aufgabe wird erfindungsgemäß gelöst durch eine Mischung umfassend oder enthaltend

a. mindestens einen solvatochromen Farbstoff,

b. mindestens ein Lösungsmittel,

c. mindestens einen Duftstoff der ausgewählt ist aus der Gruppe, die gebildet wird von ungesättigten Duftstoffen, Ketonen, Ethern, Moschusduftstoffen und Alkoholen sowie gegebenenfalls

d. mindestens ein Stabilisierungsmittel.

Überraschenderweise wurde gefunden, dass die erfindungsgemäßen Mischungen, die oben geschilderte komplexe Aufgabe in vollem Umfang lösen. Insbesondere werden die Nachteile, die mit bekannten indirekten Anzeigemechanismen verbunden sind, vermieden, denn bei zur Neige gehendem Duftstoffanteil geben die erfindungsgemäßen Systeme ein direktes visuelles Signal ab und zeichnen sich zusätzlich dadurch aus, dass sie über einen sehr langen Zeitraum lagerfähig sind und zwar auch dann, wenn diese starker Lichtbestrahlung ausgesetzt werden. Die verbesserte (Licht-)Stabilität betrifft neben den Farbstoffen auch die (Licht-)Stabilität der Duftstoffe, so dass insbesondere nach stärkerer Bestrahlung der erfindungsgemäßen Raumdüfte mit Licht sowohl ein stark wahrnehmbares Farbänderungssignal als auch ein deutlich wahrnehmbarer Wohlgeruch wahrgenommen werden kann. Die Signalfarbe des Nutzungsendes ist üblicherweise verschieden von der Farbe Weiß, der Farbwechsel ist sehr gut erkennbar und die Nutzungsendpunktanzeiger sind bevorzugt homogen gefärbt.

In einer möglichen, besonderen Ausführungsform der Erfindung soll die Maßgabe gelten, dass der Duftstoff in der erfindungsgemäßen Mischung kein Lavandinöl Abrialis ist.

**Solvatochrome Farbstoffe**

Solvatochrome Farbstoffe (Komponente a) im Sinne der vorliegenden Erfindung sind solche Farbstoffe, bei denen der Effekt der Solvatochromie auftritt. Solvatochromie ist nach der Definition gemäß Römpp Online (http://www.roempp.com/) "eine Veränderung der Lichtabsorption einer gelösten Substanz (eines Chromophors) in Abhängigkeit vom Lösemittel". Bevorzugt einzusetzende solvatochrome Farbstoffe sind ausgewählt aus Nilrot, Reichardt-Farbstoff, Dimethylaminobenzaldehyd, 4-[2-N-substitertes-1,4-hydropyridin-4-ylidine)ethylidene]cyclohexa-2,5-dien-1-on, 1-(4-hydroxyphenol)-2,4,6-triphenylpyridinium oder /und der Stoffgruppen der Merocyanine, der roten Pyrazolonfarbstoffe, Azomethinfarbstoffe, Indoanilinfarbstoffe und Pyridinium-N-phenoxidbetaine.

Bevorzugte solvatochrome Farbstoffe sind frei von Halogenatomen und Aminogruppen ($-NH_2$). Besonders bevorzugte solvatochorome Farbstoffe werden ausgewählt aus Nilrot, Reichardt-Farbstoff, 5-Dimethylamino-5'-nitro-2,2'-bithiophen oder /und der Stoffgruppe der Merocyanine.

Die solvatochromen Farbstoffe werden bevorzugt zu 0,0001 bis 0,1 Gew.-% und besonders bevorzugt zu 0,001 bis 0,01 Gew.-% in den erfindungsgemäßen Mischungen eingesetzt, jeweils bezogen auf die Summe der Komponenten a, b und c.

Die jeweilige Auswahl der speziellen solvatochromen Farbstoffe trägt zu einer gesteigerten Stabilität und insbesondere zu einer gesteigerten Stabilität der erfindungsgemäßen Mischungen bei der Bestrahlung mit hohen Lichtintensitäten bei.

**Lösungsmittel**

Lösungsmittel (Komponente b) sind insbesondere die für Duftstoffe geeignete Lösungsmittel, bevorzugt ausgewählt aus der Gruppe der Alkohole, besonders bevorzugt aus der Gruppe, die gebildet wird von Ethanol, Propanol, Butanol, Isopropylalkohol, Propandiol, Butandiol, Pentandiol, Hexandiol, Phenylethylalkohol, 3,3 Methylmethoxybutanol, Solutol, Dipropylenglykolmonomethylether, Propylenglykolphenylether, Phenoxyethylalkohol, Propylenglycol, Dipropylenglycol, Triethylenglycol, Glycerin, 2-Methyl- 1,3-propandiol .Die Auswahl der Lösungsmittel soll insbesondere ein homogenes Erscheinungsbild der erfindungsgemäßen Mischungen bzw. der erfindungsgemäßen Behälter oder / und eine besonders gute Erkennbarkeit des Farbwechsels gewährleisten.

**Duftstoffe**

Als Duftstoffe (Komponente c) werden bevorzugt solche Duftstoffe verwendet, die einen angenehmen Geruchseindruck erzeugen. Bevorzugt sind Duftstoffe aus der Gruppe, die gebildet wird von ungesättigten Duftstoffen, der Ketone, Ester, Ether, Moschusduftstoffe und Alkohole. Unter ungesättigten Duftstoffen werden im Sinne der Erfindung solche Duftstoffe verstanden, die mindestens eine Doppelbindung zwischen C-Atomen im Molekül aufweisen und sind bevorzugt einfach ungesättigte Kohlenwasserstoffe. Die jeweilige Auswahl der speziellen Duftstoffe trägt zu einer gesteigerten Stabilität und insbesondere zu einer gesteigerten Stabilität der erfindungsgemäßen Mischungen bei der Bestrahlung mit hohen Lichtintensitäten bei. Besonders deutlich wird der (licht-) stabilisierende Effekt, wenn die bevorzugten Duftstoffe mit den bevorzugten solvatochromen Farbstoffen kombiniert werden.

In einer speziellen Ausführungsform der Erfindung beträgt das Massenverhältnis der Duftstoffe zu Lösungsmitteln 0,1:1 bis 10:1, bevorzugt 0,2 :1 bis 5:1 und besonders bevorzugt 0,3:1 bis 3:1, um insbesondere eine besonders gute Erkennbarkeit des Farbwechsels zu gewährleisten.

In einer weiteren speziellen Ausführungsform der Erfindung ist der Betrag der Differenz aus dem log pOW der Lösungsmittel und der Duftstoffe mindestens 1. Der log pOW im Sinne der Erfindung wird definiert als der dekadische Logarithmus des n-Oktanol-Wasser-Verteilungskoeffizienten:

$$\log pOW = \log c_O - \log c_W$$

wobei $c_O$ die Konzentration der Lösungsmittel bzw. der Duftstoffe in der oktanolreichen Phase und $c_W$ die Konzentration der Lösungsmittel bzw. der Duftstoffe in der wasserreichen Phase ist.

Unter dem Betrag der Differenz aus dem log pOW der Lösungsmittel und der Duftstoffe B wird erfindungsgemäß der Wert

$$B = |\log pOW \text{ (Lösungsmittel)} - \log pOW \text{ (Duftstoffe)}|$$

verstanden. Bevorzugt ist B größer oder gleich 1,5, besonders bevorzugt größer oder gleich 2 und insbesondere bevorzugt größer oder gleich 2,5. Dieses bevorzugte Merkmal dient insbesondere dazu, eine besonders gute Erkennbarkeit des Farbwechsels zu gewährleisten.

In einer weiteren bevorzugten Ausführungsform der Erfindung ist die Verdunstungsrate V der Duftstoffe mindestens doppelt so groß wie die Verdunstungsrate der polaren Lösungsmittel. Unter der Verdunstungsrate V wird erfindungsgemäß der auf die Oberfläche bezogene Massenverlust innerhalb eines definierten Zeitraums unter definierten und identischen Bedingungen verstanden:

$$V = \frac{m(t0) - m(t)}{A_P * t}$$

Dabei bedeuten m (t0) die Masse der Probe zu Versuchsbeginn, m(t) die Masse der Probe nach der Verdunstungszeit t, $A_P$ die Oberfläche des Lösungsmittels bzw. der Duftstoffe und t der Zeitraum, in dem die Verdunstung stattgefunden hat. Zur Gewährleistung einer Vergleichbarkeit sind $A_P$, t und m (t0) konstant.

Als Referenz wird die folgende Methode angewendet: 40 mg der des Lösungsmittels oder der Duftstoffe werden auf eine flache Probenpfanne des DVS 1000 der Firma SMS Surface Measurement Systems UK gegeben. Die Massenverluste der Probe werden bei 40°C ermittelt. Dieses bevorzugte Merkmal dient insbesondere dazu, eine besonders gute Erkennbarkeit des Farbwechsels zu gewährleisten.

Der Membran-Permeationskoeffizient der Duftstoffe ist bevorzugt mindestens doppelt so groß wie der Membran-Permeationskoeffizient der polaren Lösungsmittel. Unter dem Membran-Permeationskoeffizienten M wird erfindungsgemäß der flächenbezogene Massenstrom der Lösungsmittel bzw. der Duftstoffe durch die semipermeable Membran verstanden.

$$M = \frac{m\,(t0) - m(t)}{A_M * t}$$

Darin bedeuten m (t0) die Masse der Probe zu Versuchsbeginn, m(t) die Masse der Probe nach der Verdunstungszeit t, AM die Oberfläche der Membran und t der Zeitraum, in dem die Verdunstung stattgefunden hat. Zur Gewährleistung einer Vergleichbarkeit sind AM, t und m (t0) konstant.

Als Referenz zur Bestimmung des Membran-Permeationskoeffizienten M wird die folgende Methode angewendet: 20g Lösungsmittel bzw. Duftstoffe werden in eine Petrischale aus Glas gegeben. Die semipermeable Membran wird so auf den Rand der Petrischale aufgeklebt, dass die Öffnung der Petrischale vollständig mit der Membran bedeckt ist und der Klebstoff die Anordnung so abdichtet, dass das Lösungsmittel bzw. die Duftstoffe nicht seitlich entweichen können. Nach 2 Wochen Lagerung bei 25 °C wird der Massenverlust und M gemäß der Gleichung bestimmt. Dieses bevorzugte Merkmal dient insbesondere dazu, eine besonders gute Erkennbarkeit des Farbwechsels zu gewährleisten.

Eine außerordentlich gute Erkennbarkeit des Farbwechsels wird erzielt, wenn die erfindungsgemäß bevorzugten Bereiche zu B, die erfindungsgemäß bevorzugten Massenverhältnisse aus Duftstoffen zu Lösungsmitteln, die erfindungsgemäß bevorzugten Verdunstungsraten V und die erfindungsgemäß bevorzugten Membranpermeationskoeffizienten M gemeinsam in einer erfindungsgemäßen Mischung und dem erfindungsgemäßen Behälter erfüllt werden.

Der pH-Wert der erfindungsgemäßen Mischung ist vorzugsweise im neutralen Bereich einzustellen. Bevorzugt beträgt der pH-Wert der erfindungsgemäßen Mischung etwa 5 bis etwa 9 und besonders bevorzugt 6 bis 8. Die Einhaltung des bevorzugten pH-Bereichs verstärkt nochmals die (licht-)stabilisierende Wirkung der erfindungsgemäßen Mischung.

Die Mischung kann optional Stabilisierungsmittel zur weiteren Erhöhung der (Licht-) Stabilität enthalten, Unter Stabilisierungsmitteln werden erfindungsgemäß bevorzugt Lichtschutzmittel, Antioxidantien und Emulgatoren verstanden.

Den erfindungsgemäßen Mischungen können gegebenenfalls weitere Farbstoffe aus der Gruppe der nicht-solvatochromen Farbstoffe hinzugefügt werden, um gegebenenfalls andere Farbnuancen oder dergleichen einzustellen.

In einer weiteren bevorzugten Ausführungsform der Erfindung liegt die erfindungsgemäße Mischung in einem Behälter vor, der eine semipermeable Membran aufweist oder aus einer semipermeablen Membran besteht. Die semipermeable Membran besteht in einer bevorzugten Ausführungsform aus Polyethylen. Weitere Formen, in denen die erfindungsgemäßen Mischungen eingesetzt werden können sind Duftbäumchen, Duftsteine, Lufterfrischer für Geschirrspülmaschinen, Wäschetrockner, Toilettenduftsteine, Duftkerzen, Duftöllampen mit transparentem Ölvorratsbehälter, Duftgele, insbesondere Duftgele enthaltend Polyamid (z.B: Sylvaclear), Polyurethan (z.B: IFO), Polysaccharide (z.B. Carragenan), mikroporöse Polymere (z.B: Accurel und Ethylvinylacetat Vinylacetat).

Die Erfindung betrifft ferner ein Verfahren zur Nutzungsendpunktsanzeige von Duftstoffsystemen, bei dem man Duftstoffe mit Lösungsmitteln und solvatochromen Farbstoffen und gegebenenfalls Stabilisatoren vermischt und der Luft und /oder einer Bestrahlung mit Licht aussetzt, wobei ein Farbumschlag im Bereich des sichtbaren Lichtes stattfindet, sobald eine hinreichende Menge an Lösungsmittel oder Duftstoff an die Luft abgegeben worden ist. Bevorzugt lassen sich die Duftstoffsysteme, bei dem man Duftstoffe mit Lösungsmitteln und solvatochromen Farbstoffen und gegebenenfalls Stabilisatoren vermischt über einen längeren Zeitraum lagern, bevor Lösungsmittel oder Duftstoff an die Luft abgegeben werden. Der Lagerzeitraum kann einen mindestens einen Tag, bevorzugt mindestens eine Woche, weiter bevorzugt mindestens einen Monat und insbesondere bevorzugt mindestens ein Jahr betragen. Die Temperatur während der Lagerung kann zwischen 0 und 50°C erfolgen, bevorzugt zwischen 15 und 30°C. Es ist dabei nicht notwendig eine konstante Temperatur einzuhalten. In der bevorzugten Ausführung der Erfindung wird der Geruch der Mischung oder/und die Farbe während der Lagerung nicht verändert, auch und insbesondere, wenn während der Lagerung eine intensive Bestrahlung mit Licht erfolgt. Zur Beurteilung einer Farb- oder/und einer Geruchsveränderung kann insbesondere der Duo-Trio Test gemäß DIN EN ISO 10399:2010-06 herangezogen werden.

Beispiele

Mischungen aus Farbstoffen, Lösungsmitteln und Duftstoffen wurden in eine quaderförmige Schale aus durchsichtigem Polyethylen gefüllt und die Schale wird mit einer 30 μm dicken Membran aus Polyethylen versiegelt. Länge, Breite und Höhe der Schale betrugen 30 x 5 x 50 mm, die Fläche der Membran beträgt 30x50 mm.

| Bsp. | Farbstoff | Lösungsmittel | Duftstoff |
|---|---|---|---|
| 1 | 0,001 Gew-% Nilrot | 20,000 Gew-% Phenoxyethylalkohol | 79,999 Gew-% Dodecanol |
| 2 | 0,002 Gew-% Reichardt Farbstoff | 30,000 Gew-% Dipropyleneglycol | 69,998 Gew-% Dibenzylether |
| 3 | 0,0015 Gew-% Dimethylaminobenzaldehyd | 99,8985 Gew-% Isopropylalkohol | 0,1 Gew-% Muscone (Moschus) |
| 4 | 0,001 Gew-% Dimethylamino-5'-nitro-2,2'-bithiophen | 80,000 Gew-% Propylenglycol | 19,999 Gew-% Filbertone (Keton) |
| V1 | 0,01 Gew-% Nilblau | 20,000 Gew-% Dipropylenglycol | 79,99 Gew-% Hexanal |
| V2 | 0,01 Gew-% Nilblau | 80,000 Gew-% Dipropylenglycol | 19,99 Gew-% Capronsäureethylester |

Die Duftsysteme wurden in einem Abstand von 0,5 m an ein Fenster gestellt, wobei sie gelegentlich der Sonnenstrahlung ausgesetzt waren. Nach 4 Wochen waren die Vergleichsversuche V1 und V2 bereits verblichen. Die erfindungsgemäßen Versuche 1 bis 4 hingegen waren noch deutlich gefärbt und zeigten nach der weiteren Lagerung und nach dem Verbrauch der Duftstoffe das Nutzungsende durch einen Farbwechsel an.

**Patentansprüche**

1. Mischungen enthaltend

   a. mindestens einen solvatochromen Farbstoff,
   b. mindestens ein Lösungsmittel,
   c. mindestens einen Duftstoff der ausgewählt ist aus der Gruppe, die gebildet wird von ungesättigten Duftstoffen, Ketonen, Ethern, Moschusduftstoffen und Alkoholen sowie gegebenenfalls
   d. mindestens ein Stabilisierungsmittel.

2. Mischungen nach Anspruch 1, **dadurch gekennzeichnet, dass** sie solvatochrome Farbstoffe (Komponente a) enthalten, die frei von Halogen- und frei von Aminogruppen sind.

3. Mischungen nach mindestens einem der Ansprüche 1 und/oder 2, **dadurch gekennzeichnet, dass** sie solvatochrome Farbstoffe (Komponente a) enthalten, die ausgewählt sind aus der Gruppe, die gebildet wird von Nilrot, Reichardt-Farbstoff, Dimethylaminobenzaldehyd, 4-[2-N-substitertes-1,4-hydropyridin-4-ylidine)ethylidene]cyclohexa-2,5-dien-1-on, 1-(4-hydroxyphenol)-2,4,6-triphenylpyridinium, 5-Dimethylamino-5'-nitro-2,2'-bithiophen und deren Gemischen.

4. Mischungen nach mindestens einem der Ansprüche 1 und/oder 2, **dadurch gekennzeichnet, dass** sie solvatochrome Farbstoffe (Komponente a) enthalten, die ausgewählt sind aus der Gruppe die gebildet wird von Merocyaninen, roten Pyrazolonfarbstoffen, Azomethinfarbstoffen, Indoanilinfarbstoffen, Pyridinium-N-phenoxidbetainen und deren Gemischen.

5. Mischungen nach mindestens einem der Ansprüche 1 bis 4, **dadurch gekennzeichnet, dass** sie Lösungsmittel (Komponente b) enthalten, die ausgewählt sind aus der Gruppe, die gebildet wird von Ethanol, Propanol, Butanol, Isopropylalkohol, Propandiol, Butandiol, Pentandiol, Hexandiol, Phenylethylalkohol, 3,3 Methylmethoxybutanol, Solutol, Dipropylenglykolmonomethylether, Propylenglykolphenylether, Phenoxyethylalkohol, Propylenglycol, Dipropylenglycol, Triethylenglycol, Glycerin, 2-Methyl- 1,3-propandiol und deren Gemischen.

6. Mischungen nach mindestens einem der vorhergehenden Ansprüche 1 bis 5, **dadurch gekennzeichnet, dass** das Massenverhältnis der Duftstoffe (Komponente c) zu Lösungsmitteln (Komponente b) 0,1:1 bis 10:1 beträgt.

7. Mischungen nach mindestens einem der vorhergehenden Ansprüche 1 bis 6, **dadurch gekennzeichnet, dass** der Betrag der Differenz aus dem log pOW der Lösungsmittel (Komponente b) und der Duftstoffe (Komponente c) mindestens 1 beträgt.

8. Mischungen nach mindestens einem der vorhergehenden Ansprüche 1 bis 7, **dadurch gekennzeichnet, dass** sie Stabilisierungsmittel (optionale Komponente d) enthalten, die ausgewählt sind aus der Gruppe, die gebildet wird von Lichtschutzmitteln, Antioxidationsmitteln, Emulgatoren und deren Gemischen.

9. Mischungen nach mindestens einem der vorhergehenden Ansprüche 1 bis 8, **dadurch gekennzeichnet, dass** sie polare Lösungsmittel und Duftstoffe enthalten und die Verdunstungsrate der Duftstoffe mindestens doppelt so groß wie die Verdunstungsrate der polaren Lösungsmittel ist.

10. Mischungen nach Anspruch 9, **dadurch gekennzeichnet, dass** der Membran-Permeationskoeffizient der Duftstoffe mindestens doppelt so groß wie der Membran-Permeationskoeffizient der polaren Lösungsmittel ist.

11. Mischungen nach einem der vorhergehenden Ansprüche 1 bis 10, **dadurch gekennzeichnet, dass** sie in einem Behälter vorliegen, der eine semipermeable Membran aufweist oder aus einer semipermeablen Membran besteht.

12. Mischungen nach Anspruch 11 **dadurch gekennzeichnet, dass** der Behälter mit Ausnahme der semipermeablen Membran aus einem synthetischen Kunststoff besteht.

13. Verfahren zur Nutzungsendpunktsanzeige von Duftstoffsystemen, bei dem man Duftstoffe, die ausgewählt sind aus der Gruppe, die gebildet wird von ungesättigten Duftstoffen, Ketonen, Ethern, Moschusduftstoffen und Alkoholen mit Lösungsmitteln und solvatochromen Farbstoffen und gegebenenfalls Stabilisatoren vermischt und der Luft aussetzt, wobei ein Farbumschlag im Bereich des sichtbaren Lichtes stattfindet, sobald eine hinreichende Menge an Lösungsmittel oder Duftstoff an die Luft abgegeben worden ist.

14. Behälter mit einem Verschluss aus einer semipermeablen Membran, der eine Mischung nach Anspruch 1 enthält.

**EP 2 630 975 A1**

Europäisches Patentamt
European Patent Office
Office européen des brevets

# EUROPÄISCHER RECHERCHENBERICHT

Nummer der Anmeldung

EP 12 00 8256

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2011/042222 A1 (HENKEL AG & CO KGAA [DE]; SUNDER MATTHIAS [DE]; MEIER FRANK [DE]; FREE) 14. April 2011 (2011-04-14) * Seiten 2-4 * ----- | 1-14 | INV. A61L9/12 A61L9/01 G01N31/22 |
| X | DE 10 2009 058078 A1 (ARIS HANDELS GMBH [DE]) 8. Juli 2010 (2010-07-08) * Absatz [0007]; Ansprüche 1-8 * * Absätze [0009] - [0013], [0024] - [0026] * ----- | 1-14 | |
| X | EP 0 309 173 A2 (JOHNSON & SON INC S C [US]) 29. März 1989 (1989-03-29) | 1,13 | |
| Y | * Seite 3, Zeile 56 - Seite 5, Zeile 11 * * Seite 4, Zeilen 12-27 * * Tabellen 1,2 * * Ansprüche 1-10 * ----- | 2-12,14 | |
| X,D | GB 2 444 702 A (GIVAUDAN SA [CH]) 18. Juni 2008 (2008-06-18) | 1,13 | |
| Y | * Seite 2, Zeile 15 - Seite 3, Zeile 25 * ----- | 2-12,14 | RECHERCHIERTE SACHGEBIETE (IPC) A61L G01N |

Der vorliegende Recherchenbericht wurde für alle Patentansprüche erstellt

| Recherchenort | Abschlußdatum der Recherche | Prüfer |
|---|---|---|
| München | 13. Juni 2013 | Michalitsch, Richard |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

8

# EP 2 630 975 A1

**ANHANG ZUM EUROPÄISCHEN RECHERCHENBERICHT
ÜBER DIE EUROPÄISCHE PATENTANMELDUNG NR.**

EP 12 00 8256

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-06-2013

| Im Recherchenbericht angeführtes Patentdokument | Datum der Veröffentlichung | Mitglied(er) der Patentfamilie | Datum der Veröffentlichung |
|---|---|---|---|
| WO 2011042222 A1 | 14-04-2011 | DE 102009045482 A1<br>EP 2453929 A1<br>US 2012193443 A1<br>WO 2011042222 A1 | 14-04-2011<br>23-05-2012<br>02-08-2012<br>14-04-2011 |
| DE 102009058078 A1 | 08-07-2010 | KEINE | |
| EP 0309173 A2 | 29-03-1989 | EP 0309173 A2<br>GB 2209942 A<br>US 4824827 A | 29-03-1989<br>01-06-1989<br>25-04-1989 |
| GB 2444702 A | 18-06-2008 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4128508 A
- WO 03031966 A1
- GB 2444702 A
- DE 102009058078
- EP 0309173 A
- WO 2009044363 A
- WO 2011042222 A